# EUROPÄISCHE PATENTSCHRIFT

(11) **EP 0 901 772 B1**
(45) Veröffentlichungstag und Bekanntmachung des Hinweises auf die Patenterteilung: **02.02.2000**
(21) Anmeldenummer: 98113880.3
(22) Anmeldetag: 24.07.1998
(51) Int. Cl.: A61B 1/267

(54) **Laryngoskop**
Laryngoscope
Laryngoscope

(30) Priorität: 09.08.1997 DE 19734591
(43) Veröffentlichungstag der Anmeldung: 17.03.1999
(73) Patentinhaber: WILLY RÜSCH AG, D-71394 Kernen-Rommelshausen (DE)
(72) Erfinder: Ilias, Wilfried, Dr., 1080 Wien (AT)
(74) Vertreter: KOHLER SCHMID + PARTNER

(56) Entgegenhaltungen:
- DE-A- 3 915 926
- US-A- 4 877 016
- US-A- 5 363 838

## Beschreibung

Die vorliegende Erfindung betrifft ein Laryngoskop mit einem Laryngoskopspatel, der mit einem Laryngoskopgriff verbunden ist.

Ein derartiges Laryngoskop ist aus der DE 39 15 926 A1 bekanntgeworden.

Bei der Intubation eines Trachealtubus tritt die grundsätzliche Schwierigkeit auf, daß der Trachealtubus nur gefahrlos in die Luftröhre (Trachea) eingeführt werden kann, wenn der intubierende Arzt sowohl die Spitze des Trachealtubus als auch den Kehlkopfeingang gleichzeitig beobachten kann. Bei der Einführung des Trachealtubus besteht die Gefahr einer Gewebetraumatisierung, da der Weg, den der Trachealtubus zu nehmen hat, nur schwer einsehbar ist. Diese Problematik wird noch dadurch erschwert, daß die anatomischen Voraussetzungen bei jedem Patienten verschieden sind. Im Bereich des Rachenraums können Intubationshindernisse auftreten, beispielsweise infolge morphologischer Variationen wie einer überhängenden Epiglottis, Tumoren oder dergleichen.

Wenn der Trachealtubus eingeführt werden soll, muß weiterhin beachtet werden, daß sich die Längsachse des Pharynx (Rachen) und die Längsachse der Trachea bei Rückenlage des Patienten nicht decken. Erst wenn der Kopf des Patienten im Nacken überstreckt ist und beispielsweise auf einem Kissen liegt, kommen diese beiden Achsen zur Deckung. Zum Intubieren reicht diese Deckung jedoch noch nicht aus, da die optische Sichtachse des intubierenden Arztes noch immer um etwa 60 Grad zu den vorgenannten, sich deckenden Längsachsen versetzt ist. Unterkiefer und Zunge des Patienten bilden nämlich ein Sichthindernis. Erst das Anheben des Zungengrundes mit einem Laryngoskop durch einen Laryngoskopspatel reduziert den Achsenversatz auf ein vertretbares Maß zur Intubation mit einem Trachealtubus. Durch den Einsatz des Laryngoskops wird der Kehlkopfeingang sichtbar, sofern nicht patientenspezifische Schwierigkeiten vorhanden sind. Der Trachealtubus kann dann von Hand am Zungengrund entlang in die Trachea eingeführt werden.

In den bereits angesprochenen, vom Normalfall abweichenden Fällen, ist der Kehlkopfeingang meist nicht zu sehen, z.B. bei eingeschränkter Mundöffnung, bei übergroßer Zunge (Makroglossie), bei Gesichtsanomalien, wie z.B. dem Pierre-Robin-Syndrom, bei eingeschränkter Beweglichkeit der Halswirbelsäule usw.

Zur Intubation in den vorgenannten Fällen wird durch die DE 39 15 926 A1 die technische Lehre vorgeschlagen, ein Endoskop zusätzlich zum Trachealtubus in den Rachenraum einzuführen. Das bekannte Endoskop wird bereits vor dem Trachealtubus unter gleichzeitiger Verwendung eines Laryngoskopspatels eingeführt. Das verwendete Endoskop ist starr ausgebildet und erstreckt sich im eingeführten Zustand in geradem Verlauf in den Rachenraum hinein. Nachteiligerweise muß das Endoskop mit der einen Hand geführt und ausgerichtet werden, während das Laryngoskop mit der anderen Hand bewegt werden kann. Zur Einführung des Trachealtubus kann das Endoskop am Laryngoskopspatel festgeklemmt werden, damit der intubierende Arzt eine Hand frei hat, um den Trachealtubus einschieben zu können. Es wird ein starres Endoskop eingesetzt, da dessen optische Qualität größer ist als die von beispielsweise flexiblen Optiken bzw. Endoskopen. Flexible Endoskope sind einerseits teuer und andererseits verhältnismäßig störanfällig.

Bei der fiber-optischen Intubation mit Hilfe des bekannten Laryngoskops wird ein einziger Lichtleiter gleichzeitig zur Ausleuchtung und Beobachtung des Rachenraums über ein mit dem Lichtleiter verbundenes Okular verwendet. Durch die Bewegung des Laryngoskops bewegt sich auch das Endoskop mit. Folglich kommt es zu unerwünschten Verschiebungen des Abbildungsbereichs, d.h. anderen Beobachtungsbereichen des Rachenraums, bzw. zu Bildstörungen. Der intubierende Arzt muß daher zusätzliche Konzentration dafür aufwenden, daß das Endoskop stets richtig innerhalb des Rachenraums plaziert und auf das Sehfeld des Arztes ausgerichtet ist.

Der vorliegenden Erfindung liegt daher das technische Problem zugrunde, den Aufbau eines Laryngoskops mit vorgesehener optischer Kontrollmöglichkeit der Laryngoskopbewegung einfacher zu gestalten, eine höhere Präzision bei der Einführung des Trachealtubus zu ermöglichen und gleichzeitig die Handhabung zu vereinfachen, damit die Intubation an sich erleichtert wird.

Dieses technische Problem wird erfindungsgemäß dadurch gelöst, daß in den Laryngoskopgriff eine Bilderfassungseinheit integriert ist.

Durch die Integration einer Bilderfassungseinheit in den Laryngoskopgriff wird im Gegensatz zum Stand der Technik kein Okular mehr benötigt, durch das der intubierende Arzt hindurchschauen muß. Der Rachenraum kann nunmehr entweder direkt über einen kleinen Monitor am Laryngoskopgriff oder über einen an die Bilderfassungseinheit anschließbaren externen Monitor beobachtet werden.

Das bekannte Laryngoskop setzt sich aus mehreren Einzelteilen zusammen, die miteinander lösbar verbunden werden können. Das erfindungsgemäße Laryngoskop ist dagegen kompakt aufgebaut und so ausgestaltet, daß alle notwendigen Komponenten zur visuellen Betrachtung des Rachenraums im Laryngoskop selbst angeordnet sind und daß es mit einer Hand präzise bedient werden kann.

Durch die Integration der Bilderfassungseinheit in den Laryngoskopgriff wird auch die Länge der Lichtleiter zwischen Bilderfassung und Bildaufnahme verkürzt. Dies trägt zu einer Qualitätserhöhung der aufgezeichneten Bilder bei. Es können keine Qualitätsverluste von der Bildaufnahme bis zur Bilddarstellung auftreten.

Bei einer bevorzugten Ausführungsform sind mindestens ein erster Lichtleiter zur Ausleuchtung eines Körperhohlraums und ein zweiter Lichtleiter, bevorzugt ein geordnetes Multifaserbündel, zur Bilderfassung vorgesehen, die einenends mit einer Lichtquelle bzw. mit der Bilderfassungseinheit koppelbar sind und sich anderenends bis in den Laryngoskopspatel erstrecken. Durch die Verwendung eines Lichtleiters zur Ausleuchtung und eines Lichtleiters zur Bilderfassung findet eine totale Trennung bzw. Entkopplung von Beleuchtung und Bilddarstellung statt. Dies beeinflußt die Bildqualität in positiver Weise. Über die Lichtleiter, aus vorzugsweise Glasfaser-Materialien, wird der Blickwinkel des intubierenden Arztes an das patientenseitige Ende des Laryngoskopspatels verlegt. Folglich ist es möglich, ein durch Zahnreihen, Unterkiefer und Lippen behindertes Blickfeld auch bei anatomischen Fehlbildungen zu umgehen. Der erste und zweite Lichtleiter können in den Laryngoskopspatel vollständig integriert sein. Der Laryngoskopspatel bildet ein Gehäuse, in dem die Lichtleiter geschützt untergebracht sind. Eine Öffnung im Bereich der Spatelspitze des Laryngoskops ermöglicht den Lichtaustritt bzw. Lichteintritt in die Lichtleiter zur Bildaufnahme aus dem Rachenraum.

Bei einer anderen Ausführungsform ist der Laryngoskopspatel, einschließlich der mit dem Laryngoskopspatel verbundenen Lichtleiter, an dem Laryngoskopgriff lösbar befestigt. Daher läßt sich der Laryngoskopspatel leicht von dem Laryngoskopgriff trennen, um eine Reinigung oder Desinfizierung des Laryngoskopspatels oder der Lichtleiter zu gestatten. Es können diejenigen Teile des Laryngoskops, die mit Körperflüssigkeiten des Patienten in Berührung kommen, separat gereinigt werden. Auf den Laryngoskopgriff können auch unterschiedliche Laryngoskopspatel mit unterschiedlicher Größe und/oder mit unterschiedlichen daran angebrachten Lichtleitern je nach Einsatzzweck befestigt werden.

Eine Weiterbildung dieser Variante besteht darin, daß der Laryngoskopspatel mit dem Laryngoskopgriff über einen arretierbaren Rastverschluß befestigbar ist. Damit wird auch eine formschlüssige Positionierung des Laryngoskopspatels zur Verkopplung der innerhalb des Laryngoskopgriffs befindlichen Optik gewährleistet. Der Laryngoskopspatel kann beispielsweise einfach eingeklinkt und über einen Bajonettverschluß arretiert werden. Daher kann stets eine optimale Bilderfassung aus dem Rachenraum gewährleistet werden.

Zur Optimierung der Bildqualität in Verbindung mit einer kompakten und platzsparenden Bauweise des Laryngoskops ist es vorteilhaft, daß die Bilderfassungseinheit eine Videokamera ist, bevorzugt eine digitale CCD-Videokamera.

Die Lichtleiter sind zur Beleuchtung an eine Kaltlichtquelle (Halogenlicht) anschließbar, um auch kleinste Hindernisse im Rachenraum ausleuchten, erfassen und beobachten zu können.

Die Handhabung des Laryngoskops läßt sich noch weiter verbessern, wenn eine der genannten Lichtquellen innerhalb des Laryngoskopgriffs untergebracht ist. Moderne leistungsstarke Lichtquellen (einschließlich einer Batterie oder eines Akkus) lassen sich aufgrund ihres geringen Platzbedarfs in den Laryngoskopgriff einbauen, so daß Verbindungsleitungen des Laryngoskopgriffs zu einer separaten Lichtquelle nicht mehr benötigt werden.

Zur Erfassung eines möglichst großen Winkelbereichs der Abbildung des Rachenraums ist der Bilderfassungseinheit innerhalb des Laryngoskopgriffs ein fokussierendes Linsensystem vorgeschaltet. Vorzugsweise kann ein Winkelbereich von ca. 60 Grad abgebildet werden. Über das Linsensystem kann von außen die Schärfe des Bildes eingestellt werden.

Das erfindungsgemäße Laryngoskop kann an bestehende Einrichtungen optischer oder elektrischer Art angeschlossen werden, wenn die Lichtleiter oder sonstigen Anschlußleitungen für die Bilderfassungseinheit an dem Patienten abgewandten Ende des Laryngoskopgriffs Konnektiereinrichtungen für weitere Verbindungsleitungen aufweisen. Der intubierende Arzt muß dann lediglich den Laryngoskopgriff in der Hand halten, während Lichtquellen und sonstige angeschlossene Einrichtungen außerhalb des Bewegungsbereichs des Arztes angeordnet sind.

Zur Auswertung erfaßter Daten und einer besseren Beobachtung des Rachenraums ist die Bilderfassungseinheit bei einer weiteren Variante der Erfindung an ein externes Bildverarbeitungssystem anschließbar. Gleichzeitig zur Beobachtung können die Daten des Patienten erfaßt werden und mit Referenzdaten verglichen und ausgewertet werden.

Eine weitere Ausführungsform des Laryngoskops weist einen Laryngoskopmonitor auf, der am Laryngoskopgriff vorgesehen ist. Der Laryngoskopmonitor kann in den Laryngoskopgriff vollständig integriert sein oder aber am Griffgehäuse befestigt sein. Durch den Laryngoskopmonitor kann der behandelnde Arzt die Behandlung des Patienten noch besser kontrollieren, ohne sich zu einem separaten Bildschirm hin drehen zu müssen. Als Laryngoskopmonitore eignen sich insbesondere kleine Flachbildschirme, bei denen die Bilddarstellung durch Lasertechnologie (TFT-LCD-Monitore) erzeugt wird.

In den Laryngoskopgriff können auch Einrichtungen integriert sein, die für eine kabellose Bedienung erforderlich sind. Das erfindungsgemäße Laryngoskop ist dann mit Akkus für die Lichtquelle, den Laryngoskopmonitor, die Bilderfassungseinheit usw. ausgerüstet. Auf elektrische Anschlußkabel kann dann verzichtet werden. Zusätzlich ist auch ein Sender im Laryngoskopgriff eingebaut, der Daten zur separaten Bilderfassungseinheit übermitteln kann. Durch diese Ausgestaltung wird es möglich, daß der behandelnde Arzt das Laryngoskop noch besser handhaben kann, da seine Bewegungsfreiheit in keinster Weise beeinträchtigt wird.

Weitere Vorteile ergeben sich aus der Beschreibung und der beigefügten Zeichnung. Ebenso können die vorstehend genannten und die noch weiter aufgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter.

Die Erfindung ist in der Zeichnung dargestellt und wir anhand eines Ausführungsbeispieles näher erläutert. Es zeigt:
- **Fig. 1**: eine schematische Darstellung der Handhabung des erfindungsgemäßen Laryngoskops;
- **Fig. 2**: einen Schnitt entlang der Längsachse des Laryngoskopgriffs des Laryngoskops nach Fig. 1.

Die Figuren der Zeichnung zeigen den erfindungsgemäßen Gegenstand stark schematisiert und sind nicht maßstäblich zu verstehen. Die gegenständlichen Merkmale sind in den einzelnen Figuren so dargestellt, daß ihr Aufbau gut gezeigt werden kann.

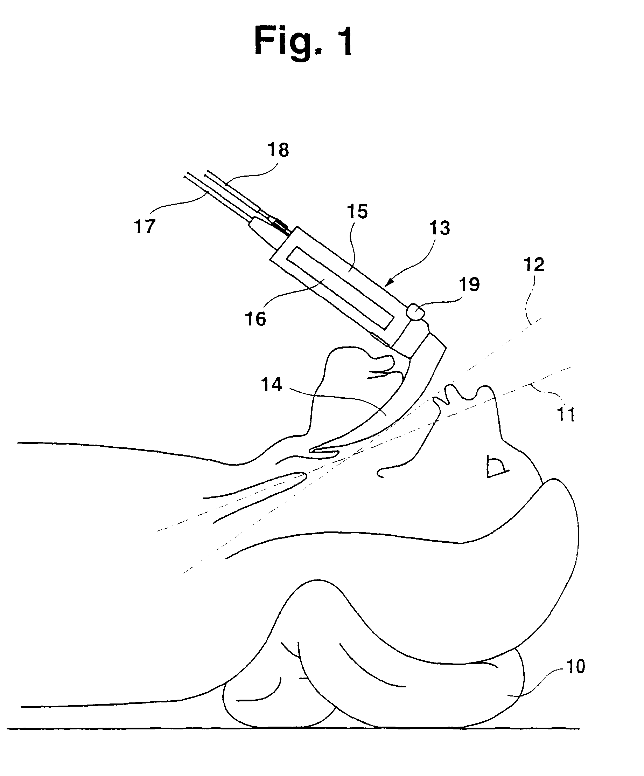

Gemäß **Fig. 1** liegt ein zur Intubation bereiter Patient auf dem Rücken. Der Kopf des Patienten ist im Nacken überstreckt und liegt auf einem Kissen 10. Durch die Überstreckung des Kopfes decken sich in der vorliegenden Stellung die Längsachse des Pharynx und die Längsachse der Trachea bzw. des Larynx (Kehlkopf). Die Deckung dieser Achsen ist mit Bezugsziffer 11 bezeichnet. Für den intubierenden Arzt ergibt sich eine optische Sichtachse 12. Die Längsachse 11 von Pharynx und Larynx ist zur optischen Längsachse 12 versetzt angeordnet. Folglich wird der Kehlkopfeingang erst dann sichtbar, wenn der Zungengrund durch das Laryngoskop 13 angehoben wird.

Das Laryngoskop 13 besteht im wesentlichen aus einem Laryngoskopspatel 14 und einem Laryngoskopgriff 15. Das Laryngoskop 13 läßt sich durch den Laryngoskopgriff 15 halten und handhaben. Der Laryngoskopspatel 14 dient dazu, den Zungengrund anzuheben und/oder zur Seite zu drücken, damit ein Trachealtubus in die Trachea eingeführt werden kann. Innerhalb des Laryngoskopspatels 14 sind in der Figur nicht sichtbare Lichtleiter angeordnet, die der Ausleuchtung des Rachenraums und gleichzeitig zur visuellen Erfassung des Rachenraums dienen. Die Lichtleiter sind innerhalb des Laryngoskopspatels 14 bis zu einer Bilderfassungseinheit 16 geführt, die innerhalb des Laryngoskopgriffes 15 angeordnet ist. Die Bilderfassungseinheit 16 (vorzugsweise eine digitale Videokamera) kann über eine Anschlußleitung 17 mit einem externen Bildverarbeitungssystem verbunden werden. Das Bildverarbeitungssystem ermöglicht es, dem Arzt in Verbindung mit einem Bildschirm oder Monitor den Rachenraum als Bild darzustellen, während der Arzt mit der einen Hand das Laryngoskop bedient und mit der anderen Hand den Trachealtubus einführt. Über einen Lichtleiter 18 ist das Laryngoskop 13 an eine Kaltlichtquelle (Halogenlichtquelle) konnektierbar.

Der Laryngoskopspatel 14 ist an dem Laryngoskopgriff 15 mit Hilfe eines Rastverschlusses 19 lösbar und abnehmbar verbunden. Aus diesem Grund kann der Laryngoskopspatel 14 mit den innerhalb des Laryngoskopspatels befindlichen Lichtleitern oder Kabeln komplett von dem Laryngoskopgriff 15 abgetrennt werden, beispielsweise um ausgetauscht oder gereinigt zu werden. Am Laryngoskopgriff 15 können unterschiedliche Laryngoskopspatel 14 mit unterschiedlichen Größen, Ausformungen und/oder mit unterschiedlichen Lichtleitern oder Kabeln fest fixiert werden. Der Rastverschluß 19 gewährleistet, daß der Laryngoskopspatel 14 derart an die Bilderfassungseinheit 16 konnektierbar ist, daß stets der für den Arzt wichtige Bereich des Rachenraums auf einer Bildschirmfläche abgebildet werden kann.

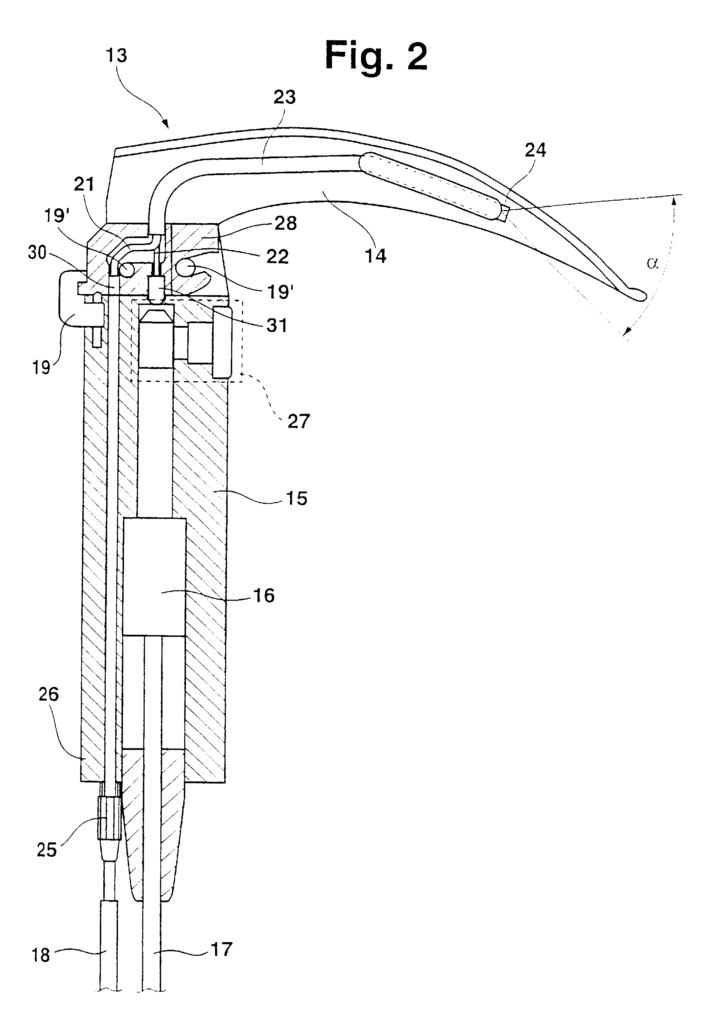

In der **Fig. 2** ist ein Längsschnitt durch das Laryngoskop 13 gezeigt. Innerhalb des Laryngoskopspatels 14 ist ein erster Lichtleiter 21 zur Ausleuchtung eines Körperhohlraums geführt. Parallel zu dem ersten Lichtleiter 21 verläuft ein zweiter Lichtleiter 22, der zur Bilderfassung vorgesehen ist. Die beiden Lichtleiter 21 und 22 verlaufen in einer Außenummantelung 23 bis in den Spitzenbereich zu einem patientenseitigen Ende 24 des Laryngoskopspatels 14. Durch die Lichtleiter 21 und 22 wird dem intubierenden Arzt ein Winkelbereich α von ca. 60 Grad als Einblick in den Rachenraum ermöglicht, der auf einem Monitor dargestellt wird. Der Lichtleiter 21 erstreckt sich durch den Laryngoskopgriff 15 hindurch bis zu einer Konnektiereinrichtung 25. Über die Konnektiereinrichtung 25 kann der Lichtleiter 21 mit einem weiteren Lichtleiter 18 verbunden werden, der an eine Lichtquelle angeschlossen ist. Auch die Anschlußleitung 17 für die Bilderfassungseinheit 16 könnte mittels einer entsprechenden Konnektiereinrichtung im Bereich des patientenabgewandten Endes 26 des Laryngoskopgriffs 15 an weitere Bildauswertungseinrichtungen anschließbar sein.

Für die Qualität der erfaßten Bilder des Rachenraums durch die Bilderfassungseinheit 16 ist es günstig, daß die Lichtleiter zur Ausleuchtung und Bilderfassung stets getrennt und voneinander entkoppelt sind. Die innerhalb des Lichtleiters 22 übermittelten optischen Signale werden durch ein Linsensystem 27 fokussiert, gefiltert und aufbereitet.

Der komplette Laryngoskopspatel 14 ist lösbar mit dem Laryngoskopgriff 15 verbunden. Die lösbare Verbindung ist realisiert durch einen Verriegelungsabschnitt 28 und Rastbolzen 29. Der Verriegelungsabschnitt 28 kann die Rastbolzen 29 hintergreifen und durch den Rastverschluß 19 in dieser Position fixiert werden. Die Lichtleiter 21 und 22 sind im Übergang von Laryngoskopspatel 14 zum Laryngoskopgriff 15 hin durch entsprechende Anschlüsse 30 und 31 im Bereich des Laryngoskopgriffs 15 überbrückbar, um die entsprechenden zu übermittelnden Signale weiterzuleiten.

Am Laryngoskopgriff 15 könnte auch ein nicht dargestellter Laryngoskopmonitor befestigt werden, auf dem die erfaßten Bilder aus dem Rachenraum dargestellt werden. Zusätzliche Einrichtungen zur Bilderfassung (Akkus, integrierte Lichtquellen etc.) und Datenübertragung (Sender) zu einer externen Bildverarbeitungsanlage lassen sich ebenfalls in den Laryngoskopgriff 15 integrieren, so daß keine Anschlußleitungen 17 am Laryngoskopgriff 15 benötigt werden.

Ein Laryngoskop 13 besitzt einen Laryngoskopspatel 14, der mit einem Laryngoskopgriff 15 verbunden ist. In den Laryngoskopgriff 14 ist eine Silderfassungseinheit 16 integriert. Der Aufbau des Laryngoskops 13 mit vorgesehener optischer Kontrollmöglichkeit der Laryngoskopbewegung ist gegenüber dem Stand der Technik einfacher gestaltet. Es kann eine höhere Präzision bei der Einführung eines Trachealtubus erreicht werden. Gleichzeitig ist die Handhabung vereinfacht, damit die Intubation an sich erleichtert wird.

## Patentansprüche

1. Laryngoskop (13)
mit einem Laryngoskopspatel (14), der mit einem Laryngoskopgriff (15) verbunden ist,
**dadurch gekennzeichnet**,
daß in den Laryngoskopgriff (15) eine Bilderfassungseinheit (16) integriert isc.

2. Laryngoskop nach Anspruch 1, dadurch gekennzeichnet, daß mindestens ein erster Lichtleiter (21) zur Ausleuchtung eines Körperhohlraums und ein zweiter Lichtleiter (22), bevorzugt ein geordnetes Multifaserbündel, zur Bilderfassung vorgesehen sind, die einenends mit einer Lichtquelle bzw. mit der Bilderfassungseinheit (16) koppelbar sind und sich anderenends bis in den Laryngoskopspatel (14) erstrecken.

3. Laryngoskop nach Anspruch 1 oder 2, dadurch gekennzeichnet, daß der Laryngoskopspatel (14), einschließlich der mit dem Laryngoskopspatel (14) verbundenen Lichtleiter (21, 22), an dem Laryngoskopgriff (15) lösbar befestigt ist.

4. Laryngoskop nach Anspruch 3, dadurch gekennzeichnet, daß der Laryngoskopspatel (14) mit dem Laryngoskopgriff (15) über einen arretierbaren Rastverschluß (19) befestigbar ist.

5. Laryngoskop nach einem der vorhergehenden Ansprüche, dadurch gekennzeichnet, daß die Bilderfassungseinheit (16) eine Videokamera ist.

6. Laryngoskop nach einem der vorhergehenden Ansprüche, dadurch gekennzeichnet, daß der erste Lichtleiter (21) zur Beleuchtung an eine Kaltlichtquelle anschließbar ist.

7. Laryngoskop nach einem der vorhergehenden Ansprüche, dadurch gekennzeichnet, daß eine Lichtquelle innerhalb des Laryngoskopgriffs (15) untergebracht ist.

8. Laryngoskop nach einem der vorhergehenden Ansprüche, dadurch gekennzeichnet, daß der Bilderfassungseinheit (16) innerhalb des Laryngoskopgriffs (15) ein fokussierendes Linsensystem (27) vorgeschaltet ist.

9. Laryngoskop nach einem der vorhergehenden Ansprüche 2-8, dadurch gekennzeichnet, daß die Lichtleiter (21, 22) oder sonstigen Anschlußleitungen (17) für die Bilderfassungseinheit (16) an dem patientenabgewandten Ende (26) des Laryngoskopgriffs (15) Konnektiereinrichtungen (25) für weitere Verbindungsleitungen aufweisen.

10. Laryngoskop nach einem der vorhergehenden Ansprüche, dadurch gekennzeichnet, daß die Bilderfassungseinheit (16) an ein externes Bildverarbeitungssystem anschließbar ist.

11. Laryngoskop nach einem der vorhergehenden Ansprüche, dadurch gekennzeichnet, daß am Laryngoskopgriff (15) ein Laryngoskopmonitor vorgesehen ist.

12. Laryngoskop nach einem der vorhergehenden Ansprüche, dadurch gekennzeichnet, daß in den Laryngoskopgriff (15) Einrichtungen integriert sind, die für eine kabellose Bedienung des Laryngoskops (13) erforderlich sind.

## Claims

1. Laryngoscope (13)
having a laryngoscope spatula (14) connected to a laryngoscope grip (15),
characterized in that
an imaging unit (16) is integrated into the laryngoscope grip (15).

2. Laryngoscope according to claim 1, characterized in that at least one first light guide (21) is provided for illumination of a body cavity and one second guide (22), preferentially an ordered multi-fiber-bundle, for imaging, which can be connected at one end to a light source and to the imaging unit (16) respectively and which extend into laryngoscope spatula (14) at the other end.

3. Laryngoscope according to claim 1 or 2, characterized in that the laryngoscope spatula (14), including the light guides (21, 22) integral with the laryngoscope spatula (14), are attached to the laryngoscope grip (15) in a detachable fashion.

4. Laryngoscope according to claim 3, characterized in that the laryngoscope spatula (14) can be attached to the laryngoscope grip (15) via a lockable catch (19).

5. Laryngoscope according to any one of the preceding claims characterized in that, the imaging unit (16) is a videocamera.

6. Laryngoscope according to one of the preceding claims, characterized in that the first light guide (21) can be connected to a cold light source for illumination.

7. Laryngoscope according to any one of the preceding claims, characterized in that a light source is accommodated within the laryngoscope grip (15).

8. Laryngoscope according to any one of the preceding claims, characterized in that a focussing lens system (27) is disposed upstream of the imaging unit (16) within the laryngoscope grip (15).

9. Laryngoscope according to any one of the preceding claims 2 to 8, characterized in that the light guides (21, 22) or other connecting leads (17) for the imaging unit (16) have connecting devices (25) for additional connecting leads on that end (26) of the laryngoscope grip (15) facing away from the patient.

10. Laryngoscope according to any one of the preceding claims, characterized in that the imaging unit can be connected to an external imaging processing system.

11. Laryngoscope according to any one of the preceding claims, characterized in that a laryngoscope monitor is provided for on the laryngoscope grip (15).

12. Laryngoscope according to any one of the preceding claims, characterized in that devices are integrated into the laryngoscope grip (15) to provide for wire-less operation of the laryngoscope (13).

## Revendications

1. Laryngoscope (13) comportant une spatule de laryngoscope (14), qui est reliée à une poignée de laryngoscope (15), caractérisé en ce qu'une unité d'acquisition d'images (16) est intégrée dans la poignée de laryngoscope (15).

2. Laryngoscope selon la revendication 1, caractérisé en ce qu'il est prévu au moins un premier conducteur de lumière (21) pour éclairer une cavité corporelle et un second conducteur de lumière (22), de préférence un faisceau à fibres multiples rangé, pour l'acquisition d'images, qui peuvent être couplés à une extrémité à une source lumineuse ou à l'unité d'acquisition d'images (16) et qui s'étendent à l'autre extrémité jusque dans la spatule de laryngoscope (14).

3. Laryngoscope selon la revendication 1 ou 2, caractérisé en ce que la spatule de laryngoscope (14), y compris les conducteurs de lumière (21, 22) reliés à la spatule de laryngoscope (14), est fixée de manière amovible à la poignée de laryngoscope (15).

4. Laryngoscope selon la revendication 3, caractérisé en ce que la spatule de laryngoscope (14) peut être fixée à la poignée de laryngoscope (15) par un dispositif de verrouillage à crans (19) qui peut être bloqué.

5. Laryngoscope selon l'une des revendications précédentes, caractérisé en ce que l'unité d'acquisition d'images (16) est une caméra vidéo.

6. Laryngoscope selon l'une des revendications précédentes, caractérisé en ce que le premier conducteur de lumière (21) peut être raccordé à une source de lumière froide pour l'éclairage.

7. Laryngoscope selon l'une des revendications précédentes, caractérisé en ce qu'une source lumineuse est disposée à l'intérieur de la poignée de laryngoscope (15).

8. Laryngoscope selon l'une des revendications précédentes, caractérisé en ce qu'un système de lentilles de focalisation (27) est monté en amont de l'unité d'acquisition d'images (16) à l'intérieur de la poignée de laryngoscope (15).

9. Laryngoscope selon l'une des revendications 2-8 précédentes, caractérisé en ce que les conducteurs de lumière (21, 22) ou d'autres conduites de raccordement (17) pour l'unité d'acquisition d'image (16) comportent à l'extrémité (26) de la poignée de laryngoscope (15) distante du patient des dispositifs de connexion (25) pour d'autres conduites de liaison.

10. Laryngoscope selon l'une des revendications précédentes, caractérisé en ce que l'unité d'acquisition d'images (16) peut être raccordée à un système de traitement d'images externe.

11. Laryngoscope selon l'une des revendications précédentes, caractérisé en ce qu'un moniteur de laryngoscope est prévu sur la poignée de laryngoscope (15).

12. Laryngoscope selon l'une des revendications précédentes, caractérisé en ce que des dispositifs qui sont nécessaires pour une utilisation sans fil du laryngoscope (13) sont intégrés dans la poignée de laryngoscope (15).
